# FASCICULE DE BREVET EUROPEEN

(11) **EP 0 805 648 B1**
(45) Date de publication et mention de la délivrance du brevet: **10.05.2000**
(21) Numéro de dépôt: 96901866.2
(22) Date de dépôt: 26.01.1996
(51) Int. Cl.: A61B 5/00, A61B 5/0478, A61F 2/02

(54) **DISPOSITIF DE CONNEXION ELECTRIQUE TRANSCUTANEE POUR APPAREIL MEDICAL IMPLANTABLE**
TRANSKUTANE ELEKTRISCHE VERBINDUNGSEINHEIT FÜR EIN MEDIZINISCHES IMPLANTAT
TRANSCUTANEOUS ELECTRICAL CONNECTION DEVICE FOR MEDICAL IMPLANT APPARATUS

(30) Priorité: 26.01.1995 FR 9500882
(43) Date de publication de la demande: 12.11.1997
(73) Titulaire: Sabin, Pierre, 76230 Bois-Guillaume (FR)
(72) Inventeur: Sabin, Pierre, 76230 Bois-Guillaume (FR)
(74) Mandataire: Moncheny, Michel
(86) Numéro de dépôt international: FR9600141
(87) Numéro de publication internationale: WO9622727

(56) Documents cités:
- EP-A- 0 281 047
- CA-A- 1 065 969
- US-A- 3 870 832
- US-A- 4 186 749
- US-A- 4 471 786
- US-A- 4 495 917
- PROCEEDINGS OF THE ANNUAL INTERNATIONAL CONFERENCE OF THE IEEE ENGINEERING IN MEDICINE AND BIOLOGY SOCIETY, vol. 14, 29 Octobre 1992 - 1 Novembre 1992, PARIS (FR), pages 2672-2673, XP000347069 K. VAN SCHUYLENBERGH E.A.: "Monitoring Orthopaedic Implants Using Active Telemetry"

## Description

La présente invention concerne l'appareillage médical. Elle a pour objet un dispositif ou équipement qui, au moins suivant ses modes de réalisation préférés, trouve application pour le diagnostic, la prophylaxie et/ou le traitement de différents dysfonctionnements que l'on peut observer dans l'organisme animal ou humain.

Elle vise essentiellement à permettre d'équiper l'organisme d'un dispositif trans-cutané implanté conçu pour assurer des connexions électriques à travers la peau. Avec un tel dispositif les signaux électriques peuvent être transmis à travers la peau, à partir de capteurs implantés de manière permanente dans l'organisme, pour être exploités par des équipements extérieurs de traitement des informations portées par ces signaux. Les informations des capteurs sont ainsi rendues disponibles sans subir les déperditions qu'entraînent les tissus conjonctifs qui sont à traverser dans le cas de capteurs extérieurs apposés sur la peau, ne serait-ce même que l'épiderme. On peut aussi transmettre de l'extérieur un courant électrique d'alimentation d'un appareil électromécanique implanté à l'intérieur du corps humain, notamment pour recharger une pile et/ou batterie que le malade pourra garder en permanence sans avoir à se faire opérer pour le changer périodiquement.

Le document US-A-3,870,832 décrit un ensemble d'appareil médical selon le préambule de la revendication 1.

Par ailleurs, US-A-4,495,917 décrit un appareil qui n'est pas un dispositif trans-cutané.

L'invention propose un dispositif d'appareillage médical caractérisé en ce qu'il comporte un dispositif trans-cutané comportant un socle pourvu de moyens de fixation sur un os de l'organisme et des moyens de liaison étanche avec alternativement un bouchon temporaire et un pilier trans-cutané à l'intérieur duquel se situe une fiche mâle de connexion électrique, complémentaire d'une fiche femelle de connexion électrique incorporée dans ledit socle, et en ce que ledit socle comporte une membrane de protection étanche bactériologique de ladite fiche femelle, perforable par au moins des broches électriquement conductrices que comportent lesdits moyens de connexion électriques.

Grâce à cette conception, le dispositif trans-cutané de connexion de l'invention est facile à mettre en place en préservant la qualité du contact électrique entre fiche mâle et fiche femelle. Lors d'une première opération chirurgicale, on fixe sur un os le socle venu d'usine équipé de sa membrane de protection bactériologique et de son bouchon temporaire, lequel assure en particulier l'étanchéité au sang. Après un délai suffisant pour permettre la stabilisation du socle par ostéointégration, la membrane de protection bactériologique assure l'étanchéité lors de la deuxième phase de mise en place au cours de laquelle on remplace le bouchon temporaire par le pilier trans-cutané venu d'usine équipé de sa fiche mâle. Ce dernier est rendu définitivement solidaire du socle, par exemple par une vis axiale, dans la position assurant la liaison étanche et les connexions électriques entre fiches par perforation de la membrane.

On comprendra d'autre part de la description qui suit que, suivant les modes de réalisation et mise en oeuvre de l'invention adaptés à chaque cas d'application particulier, les différentes fonctions des éléments essentiels de l'invention peuvent être assurés par des moyens équivalents, souvent même préférentiels en relation avec les impératifs de la pratique industrielle.

On y verra en particulier que les notions de fiche femelle et fiche mâle ne sont pas limitatives et qu'elles n'impliquent pas toujours nécessairement une action physique de pénétration mécanique d'une broche ou fiche dite mâle dans une broche ou fiche dite femelle. On y verra aussi que, suivant les modes de réalisation à appliquer préférentiellement en liaison avec les cas d'application pratique particuliers, les éléments assurant les connexions électriques suivant l'invention, peuvent être prévus, à chaque fois en partie, soit sur le socle implanté lors de la première intervention chirurgicale, soit au sein du pilier mis en place au cours de la deuxième intervention chirurgicale, soit encore dans une prise amovible qui vient remplacer temporairement, entre deux séries d'essais médicaux, un capuchon obturant au repos le pilier trans-cutané assurant la liaison vers les autres éléments du dispositif suivant l'invention implantés à demeure dans l'organisme.

Suivant certains des modes de réalisation préférés de l'invention, les moyens de connexions électriques peuvent être fixés à demeure pour partie dans le socle implanté en sous-cutané dans l'organisme, pour partie dans le pilier mis en place par une seconde opération chirurgicale pour accessibilité permanente de l'extérieur, et/ou pour partie dans une prise coopérante jouant un rôle propre dans les connexions électriques à travers le pilier.

L'ensemble d'appareillage médical à implant suivant l'invention présente entre autres des particularités technologiques liées à la réalisation concrète des connexions électriques, telles qu'elles seront plus précisément décrites et revendiquées ci-après. On constatera en particulier que les notions de fiches mâles et fiches femelles n'impliquent pas nécessairement unee pénétration de l'une dans l'autre, et que des éléments suivant l'invention remplissant leurs fonctions respectives peuvent être intégrés dans ou associés à des éléments essentiels différents de l'appareillage de l'invention, suivant des choix qui prendront en compte les considérations de la pratique industrielle.

On décrira maintenant plus en détail, avec diverses variantes, une forme de réalisation particulière de l'invention qui en fera mieux comprendre les caractéristiques essentielles et les avantages, étant entendu toutefois que cette forme de réalisation est choisie à titre d'exemple et qu'elle n'est nullement limitative.

Sa description est illustrée par les figures 1 à 6 des dessins annexés, dans lesquels :
- la figure 1 représente un schéma d'ensemble d'un dispositif selon l'invention associé à des unités de traitement des signaux ;
- la figure 2 représente les différents éléments constituants du dispositif implanté trans-cutané de l'appareillage de l'invention en vue éclatée d'une coupe longitudinale ;
- la figure 3 montre une vue de dessus schématique du socle faisant partie de ce dispositif ;
- la figure 4 montre schématiquement le même socle du dispositif trans-cutané dans sa liaison avec un implant sous-cutané ;
- la figure 5 correspond à la figure 4 avec l'implant sous-cutané terminé ;
- la figure 6 est une vue partielle de dessus d'un tel implant sous-cutané ;
- la figure 7 illustre, dans une vue éclatée, un second mode de réalisation de l'invention ;
- la figure 8 montre en coupe longitudinale le dispositif de la figure 7 au premier stade des opérations d'implantation ;
- la figure 9 montre le dispositif des figures 7 et 8, en illustrant son raccordement avec une prise de raccordement électrique extérieure à l'organisme ;
- la figure 10 illustre de même le dispositif quand l'individu porteur n'est pas en situation d'observation, la prise 72 de la figure 9 étant alors remplacée par un capuchon protecteur 71.

Pour des raisons de clarté, les mêmes éléments ont été désignés par les mêmes références et la représentation des dessins est schématique.

Dans un mode de réalisation particulier décrit, qui constitue une forme de mise en oeuvre particulièrement avantageuse de l'invention, l'appareillage médical, objet de celle-ci, est destiné à capter des informations transmises par courant électrique en permanence d'un organe du corps humain, tel que le crâne pour un électroencéphalogramme ou le myocarde pour un électrocardiogramme, à partir de récepteurs implantés dans le corps. Il est toutefois également utilisable, simultanément ou séparément, pour alimenter en courant électrique ou commander un récepteur similairement implanté, comme peuvent l'être un stimulateur cardiaque ou tout autre dispositif électromécanique.

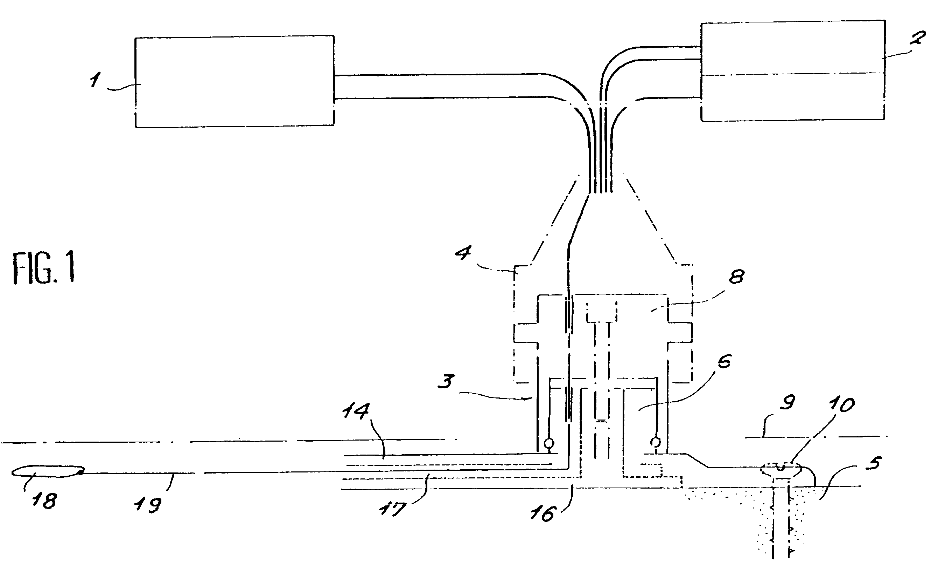

C'est pourquoi on a fait apparaître schématiquement sur la figure 1 des équipements extérieurs au corps, que sont notamment une unité 1 de traitement de signaux électriques provenant de capteurs appropriés qui les recueillent dans le corps et les transmettent à l'unité 1 à travers le dispositif trans-cutané de l'invention, et une unité 2 d'alimentation ou recharge d'une pile ou batterie également implantée dans le corps considéré. Toutes les connexions électriques nécessaires au fonctionnement de tels équipements sont assurées à travers un dispositif trans-cutané 3 associé (ou implant juxta-osseux), réalisé suivant l'invention, grâce à un connecteur intermédiaire extérieur 4 qui est amovible.

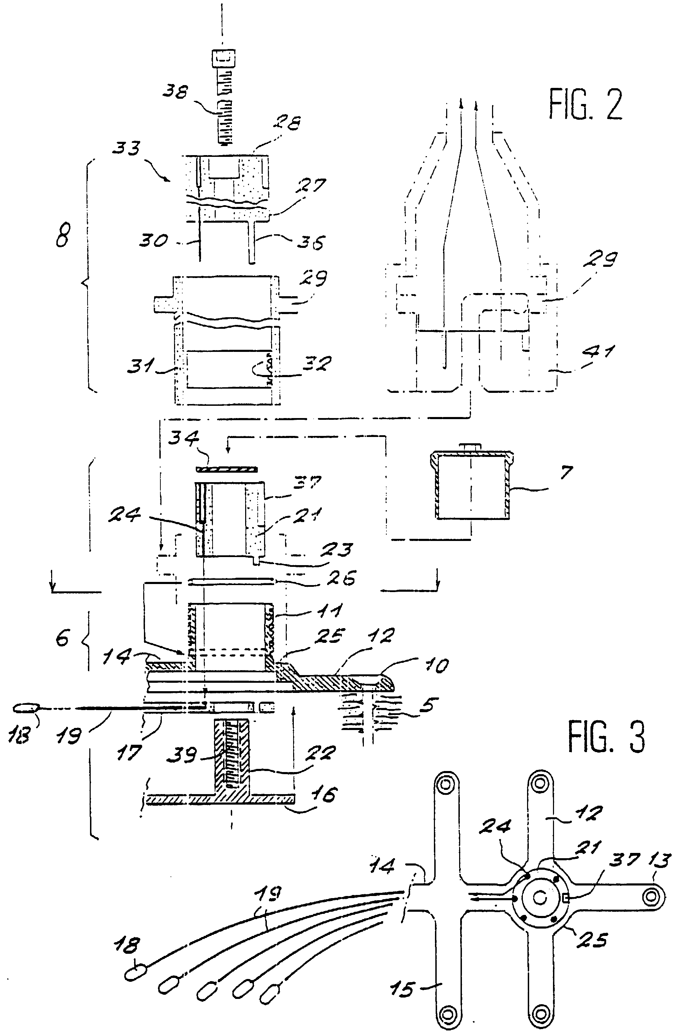

Le dispositif trans-cutané 3 est mis en place, conformément à l'invention, par un procédé de mise en oeuvre qui comporte deux phases chirurgicales. La première consiste à fixer de manière définitive, sur un os 5 du corps relativement proche de la peau, un socle 6 faisant partie du dispositif de l'invention, cependant qu'il est fermé par un bouchon temporaire 7 (figure 2). Au cours de la seconde phase, un fois le socle stabilisé par ostéointégration, on remplace le bouchon temporaire 7 par un pilier trans-cutané 8. C'est sur ce dernier que viendront se brancher, aux moments opportuns, les unités 1 et 2, par l'intermédiaire du connecteur extérieur amovible 4, pour être ainsi mises en relation électrique à travers la peau avec les capteurs et autres équipements implantés de manière définitive à l'intérieur du corps.

Le bouchon temporaire 7 comme le pilier 8 sont réalisés suivant différents modèles, avantageusement interchangeables sur un même socle 6, de sorte que l'ensemble soit aisément adaptable à différentes longueurs suivant la profondeur de l'os d'implantation par rapport à la peau et à l'épaisseur de tissus biologiques à traverser de ce fait. De ce point de vue, et notamment pour une application à des électroencéphalogrammes périodiques, on appréciera particulièrement de fixer le dispositif trans-cutané sur le crâne, juste derrière l'oreille, de sorte que la partie apparente en soit cachée par le pavillon de celle-ci.

Le socle 6, vu de dessus comme le montre la figure 3, forme autour d'un fût central 11 des ailes de fixation radiales 12 comportant à leur bout un trou 13 dans lequel se place la tête fraisée d'une vis de fixation à l'os 10 (figures 1 et 2), plus un bras radial 14 présentant également deux ailes de fixation analogues 15. Les ailes 12 et 15 et le bras 14 constituent la base du socle, laquelle est réalisée soit, de préférence, en titane pur d'une seule pièce avec le fût 11, soit en une autre matière biocompatible, telle que les résines organiques à base de silicones. Le fût 11 présente par exemple un diamètre de l'ordre de 6 mm.

Sous ladite base, à l'opposé du fût 11, il vient s'encastrer une semelle 16, également en titane, qui est soudée en usine avec la base du socle et qui coopère avec celle-ci pour enfermer une platine 17 tout le long du bras 14.

La platine 17 constitue le support de conducteurs électriques longitudinaux, réalisés sous forme de circuits imprimés. Chacun des différents conducteurs, qui seront par exemple au nombre de 5, 7 ou 9, se termine sur la face supérieure de la platine 17 à l'intérieur du fût 11, cependant qu'à son autre extrémité, il lui est soudé un fil conducteur autonome, qui sera notamment un fil d'or gainé d'une matière biocompatible telle qu'une résine de silicone. Les différents fils conducteurs 19 partent ainsi en faisceau de l'extrémité du bras 14 vers des éléments capteurs et/ou récepteurs de signaux électriques tels que le capteur sous cutané qui a été représenté en 18 sur la figure 1.

Egalement venu d'usine avec le socle 6, on observe, conformément à la figure 2, un connecteur femelle 21, réalisé sous forme d'une pièce cylindrique annulaire qui se place sans jeu dans l'espace compris entre la face interne du fût 11 et la face externe d'un axe cylindrique 22 solidaire de la semelle 16. Dans l'ensemble monté, la fiche de connexion ou connecteur 21 vient en butée sur la platine 17 qui entoure l'axe 22. Son positionnement angulaire est assuré par un téton 23 par rapport à la platine 17, de telle sorte que les extrémités des conducteurs électriques du bras 14 viennent exactement en correspondance et en contact chacun à chacun avec les conducteurs électriques 24 de la fiche 21. Les conducteurs en circuits imprimés sont ainsi individuellement en liaison électrique avec les broches de la fiche femelle.

Sous sa forme venue d'usine, le socle 6 est fermé par un bouchon 7 qui est fixé sur un filetage externe du fût 11 jusqu'à venir buter sur le dessus du socle au niveau de l'anneau 25. Un joint torique 26 autour du fût assure l'étanchéité en cet endroit. Le bouchon 7 ne vient pas nécessairement en contact avec l'extrémité supérieure du fût 11 et de la fiche 24. Il peut au contraire être prévu que ce bouchon puisse exister en différentes longueurs tout comme le pilier trans-cutané 8 que l'on décrira maintenant.

Comme on peut le voir de la figure 3, le pilier 8 est essentiellement constitué par une bague cylindrique 31 qui, par un filetage interne 32, vient se visser sur le fût 11 du socle à la place du bouchon temporaire 7. Le joint 26 remplit alors la même fonction d'étanchéité que ci-dessus, à un moment où les saignements sont faibles lors de la deuxième phase chirurgicale de mise en place.

La bague 31 contient un prolongateur 33, de forme générale cylindrique, monté en usine de manière coulissante démontable avec elle. Dans la position représentée, ce prolongateur forme en partie inférieure une fiche mâle 27 complémentaire de la fiche femelle 21 du socle.

Lors de la deuxième phase de la mise en place du dispositif implanté, après vissage de la bague 31, les broches électriques 30 de la fiche mâle du prolongateur 33 se placent automatiquement en regard des alésages correspondants de la fiche 21 jusqu'à venir en contact électrique avec les conducteurs correspondants 24, grâce à une broche de positionnement 36, en matière non conductrice, qui, avec une longueur un peu supérieure à celle des broches conductrices 30, pénètre dans une cannelure 37 creusée à cet effet sur la fiche 21.

C'est au cours de cette opération que la broche de positionnement 36, tout comme les broches 30, perfore une membrane d'étanchéité 34 qui se trouve collée d'origine sur la face d'extrémité supérieure de la fiche 21.

Cette membrane a été représentée sur la figure 2 dans le cas où elle recouvre seulement la fiche 21. Mais l'on préfèrera souvent qu'elle déborde de cette dernière jusqu'à être collée également en recouvrement de la face extrême annulaire du fût 11 du socle, plutôt que de se reposer seulement sur le bouchon temporaire pour assurer l'étanchéité de la fiche 21 dans le socle. On peut observer par contre, conformément aux figures, que dans ce cas, la membrane 34 ayant pour rôle essentiel de protéger les conducteurs 24, elle est avantageusement perforée d'un trou face à la cannelure 37, de telle sorte que la position de cette cannelure est apparente pour faciliter le positionnement radial approprié de la fiche mâle 27 lors de la mise en place du pilier 8 équipé du prolongateur 33.

Les deux parties du dispositif trans-cutané sont définitivement fixées l'une à l'autre par une vis 38 (figure 2) qui, passant à travers un alésage axial du prolongateur 33, perce elle aussi la membrane 34 et se visse dans un alésage fileté 39 de la semelle 16.

Le prolongateur 33, qui forme la fiche mâle coopérant par la fiche femelle 21 du socle, joue en fait le rôle d'un prolongateur de connexions électriques, dans la mesure où il forme également fiche femelle dans sa partie supérieure 28 , afin d'y relier les unités extérieures 1 et 2 par tous moyens de connexion à broches mâles connus en soi. Ces derniers sont incorporés dans un boîtier de branchement 41 (figure 2) qui vient s'adapter de manière démontable sur la bague 31 du pilier trans-cutané grâce, dans le cas particulier illustré, à un système de type baïonnette illustré en 29 par des ailettes extérieures de la bague 31 du pilier.

Un implant sous-cutané destiné à compléter le dispositif suivant l'invention a été représenté sur la figure 1 sous la forme d'un capteur 18 en soi classique, sensible à l'émission d'énergie électromagnétique en surface d'un muscle ou autre tissu mou de l'organisme. Le fil d'or 19 y est soudé par son extrémité dénudée opposée au dispositif trans-cutané 3 avant la mise en place de l'ensemble.

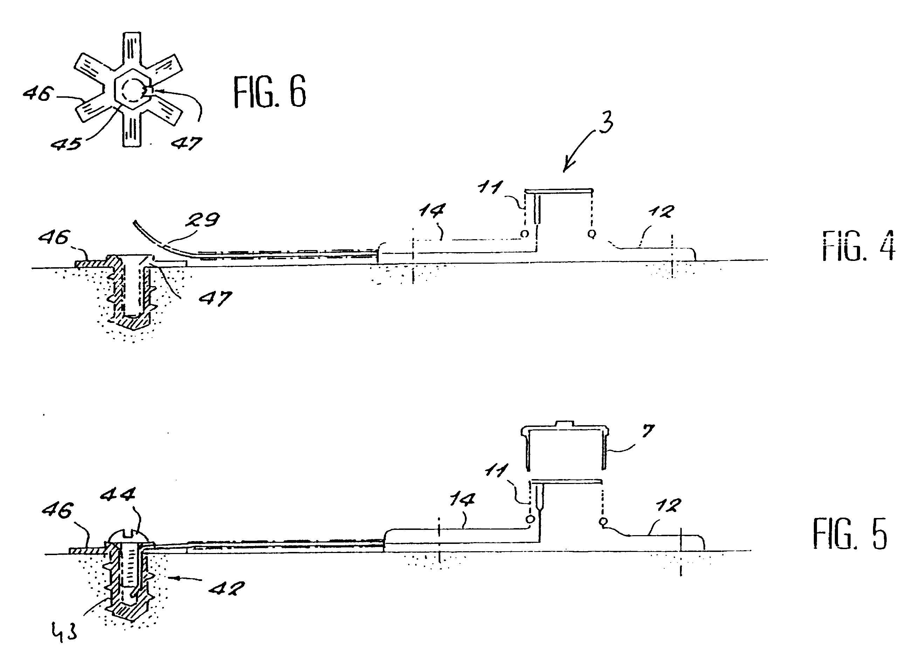

On a représenté sur les figures 4 et 5 un autre type de capteur que l'on préfèrera souvent utiliser en combinaison avec l'implant 3, pour une application notamment à la surveillance des maladies épileptiques par encéphalogrammes. Il s'agit alors d'implants sous-cutanés qui sont immobilisés par fixation sur un os tel que l'os du crâne, en des points convenablement choisis par le médecin.

Un tel implant capteur 42 est essentiellement constitué par une vis 43, présentant extérieurement un filetage du type d'un implant extra-oral, ici intra-osseux, et intérieurement un alésage fileté axial destiné à recevoir une vis de couverture 44, à travers un écrou 45 terminant la vis d'implant 43. L'extrémité dénudée du fil d'or 29 est coincée en contact conducteur électrique entre la vis d'implant 43 et la vis de couverture 44. On peut prévoir une cannelure destinée à recevoir le fil dans l'alésage fileté interne de l'implant. Ce dernier comporte en outre une rondelle à ailettes inclinées sur l'axe 46, participant à maintenir l'implant dans l'os, en évitant son dévissage. Une rainure radiale 47, en surface de cette rondelle, livre passage au fil 29. Ce dernier sort par là de l'implant sous-cutané 42 vers le dispositif trans-cutané 3. On peut aussi prévoir une gorge hélicoïdale sur le filetage externe de la vis 43 pour augmenter la surface d'interface os/implant.

Les applications typiques du dispositif d'appareillage médical de l'invention, une fois mis en place le dispositif trans-cutané 3 relié électriquement aux différents capteurs sous-cutanés 18 ou 42, passent avantageusement par un procédé d'examen médical des manifestations d'activité physiologique de l'organisme, éventuellement pathologiques, qui consiste à détecter les phénomènes électromagnétiques qu'elles induisent au moyen de capteurs directement implantés à l'intérieur du corps, et à les transmettre sous forme de signaux électriques jusqu'à l'extérieur.

Le traitement des signaux est en soi classique, mais les résultats en sont plus fins et plus fiables que tout ce qui avait pu être obtenu par le passé, si bien que par exemple, l'appareil dans son ensemble peut servir à exploiter des influx nerveux ou des efforts musculaires détectés in situ pour commander une prothèse extérieure amovible à articulations électromécaniques.

En d'autres termes, un procédé d'examen médical faisant application de l'appareillage suivant l'invention se caractérise en ce que l'on utilise des capteurs directement implantés dans le corps pour détecter des phénomènes électromagnétiques induits par des manifestations d'activité physiologique, sous forme de signaux électriques que l'on transmet jusqu'à l'extérieur du corps à travers un dispositif trans-cutané permanent comportant un socle implanté immobilisé par fixation sur un os, solidaire d'un pilier restant en permanence accessible de l'extérieur à travers la peau.

Naturellement, l'invention n'est pas limitée par les particularités qui ont été spécifiées dans ce qui précède ou par les détails du mode de réalisation particulier choisi pour illustrer l'invention. En particulier, une variante utile consisterait à remplacer les fils et broches conducteurs décrits, purement électriques, par des conducteurs électro-optiques, en substituant au circuit imprimé des fibres optiques noyées dans la platine 17, dans un système de détection opto-électronique.

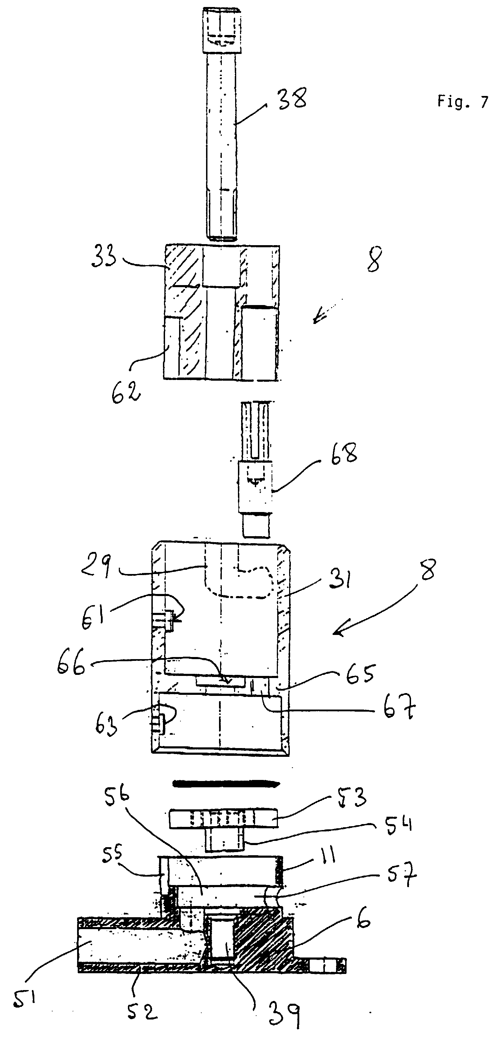

Dans la variante de réalisation illustrée par la figure 7, on observe que la base du socle 6 et la semelle 16 sont constituées d'une seule pièce. On évite aussi d'y ajouter la platine 17, les fils et circuits conducteurs étant directement mis en place dans l'espace creux 51 ménagé à cet effet dans un bras spécial 52 du socle 6, jusqu'à venir aboutir individuellement dans les perforations d'une platine annulaire 53 à laquelle ils sont fixés.

La platine 53 se monte dans le socle 6 à l'intérieur du volume du fût 11 à l'aide d'une vis centrale. Sa position finale est prédéfinie en orientation angulaire dans le fût 11, lequel présente une rainure 55 de centrage du pilier trans-cutané ultérieur. Sa position axiale est limitée lors de son vissage par une butée circulaire du socle qui ménage sous elle un espace 56 de passage des fils conducteurs, présentant un trou radial d'accès 57.

Comme dans la variante de réalisation de la figure 2, alternativement le bouchon temporaire 7 ou le pilier trans-cutané 61 viennent s'engager sur le fût 11 jusqu'à buter sur un anneau 25 en surépaisseur du socle 6 autour du fût 11. Tant qu'il s'agit du bouchon temporaire, les contacts électriques en bout des fils conducteurs dans la platine 53 sont isolés par la membrane d'étanchéité 34.

On observe donc que, suivant un mode de mise en oeuvre de l'invention qui sera souvent préféré pour la pratique industrielle, principalement pour réduire les coûts de fabrication par simplification des pièces et de leur montage sans gêner pour autant la commodité et la sécurité d'emploi pour les chirurgiens et les médecins, que les fonctions de la fiche femelle 21 de la figure 1 dans les connexions électriques avec les circuits implantés dans l'organisme, sont assurées, en mieux, par les contacts électriques accessibles en surface de la platine 53. Cette dernière combine donc différentes fonctions qui, dans la variante précédente relevaient pour partie de la fiche femelle 21 et pour partie de la platine 17, alors que la réalisation mécanique du socle et de son raccordement avec les autres éléments du dispositif est également simplifiée.

Le mode de réalisation illustré par les figures 7 à 10 ne s'intéresse pas à cette variante, mais plutôt à des modes de réalisation de l'invention particulièrement avantageux, séparément ou en leurs combinaisons opérantes, pour ce qui concerne principalement le socle à implanter dans l'organisme, le pilier trans-cutané et les éléments assurant les connexions électriques, de manière fiable sous préservation des conditions primordiales d'absence de contamination biologique.

Alors que l'on retrouve sur la figure 7 la vis 38 dans les fonctions qui ont été décrites précédemment et que l'on retrouve également le dispositif de reccordement à baïonnette 29 (sauf que les formes coopérantes les unes en protubérance, les autres en creux, sont interversées) on observe que la bague 31 du pilier 8 présente intérieurement des tétons de centrage, respectivement 61 pour coopérer avec une rainure 62 du prolongateur 33 et 63 pour coopérer avec la rainure 55 du fût 11 qui a déjà été mentionné. Ces différentes particularités assurent un positionnement angulaire prédéfini des différents éléments intervenant dans les connexions électriques essentielles à la mise en oeuvre du dispositif de l'invention.

On observe d'autre part qu'à l'intérieur de la bague 31, il est constitué, d'une seule pièce avec elle, une entretoise 65 constituant fiche de connexion électrique. Cette entretoise 65 comporte un alésage perforant central 66 qui permet la traversée de la vis 38 et qui permet également un positionnement prédéterminé du prolongateur 33 sur un épaulement interne. D'autre part, l'entretoise 65, dans sa zone annulaire autour de l'alésage central 66, est percée de trous 67 dans lesquels se positionnent des broches de connexion électrique 68 mises en place préalablement dans le prolongateur 33. Grâce à la coopération de tétons de centrage 61 et 63 avec les rainures coopérantes 62 du prolongateur 33 et 55 du fût 11 du socle 6, les broches 68 se placent exactement à travers les trous 67, en regard des contacts électriques terminant les fils de transmission au sein de la platine 53.

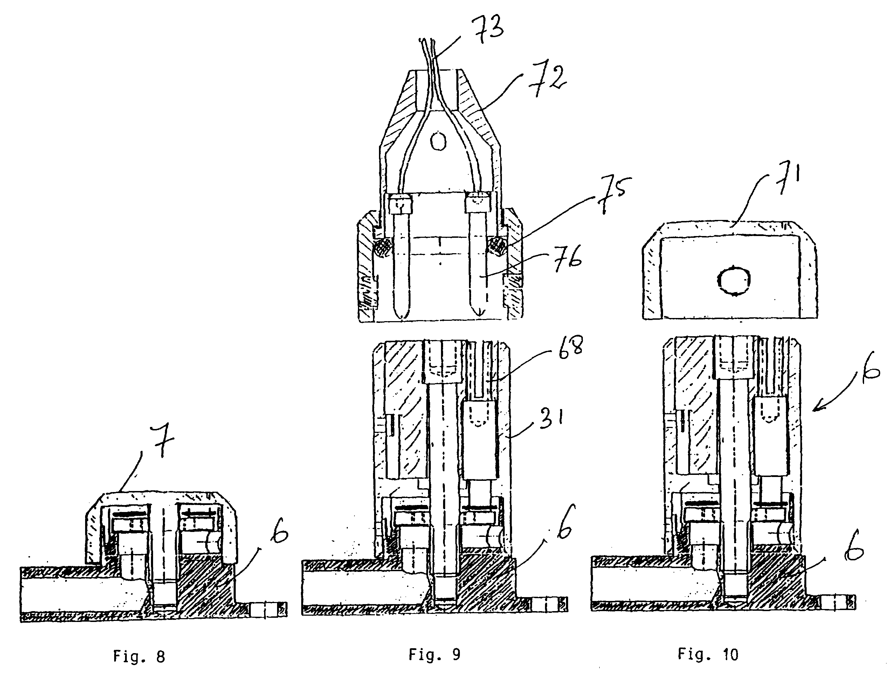

Les figures 9 et 10 illustrent comment un capuchon 71 de fermeture du dispositif trans-cutané, adapté sur le pilier 8, à l'extérieur de l'organisme, peut être remplacé par une prise 72. L'un comme l'autre se fixent sur la bague 31 du pilier 8, par vissage sur un filetage externe prévu à cet effet ou par un dispositi à hayonnette. La prise 72 est traversée par des fils 73, à utiliser pour la transmission d'informations ou d'ordres et/ou pour la réception de données émises dans l'organisme vers les dispositifs d'analyse extérieurs. La prise 72 intègre, comme il est illustré sur la figure 9, non seulement un joint 75 d'étanchéité du raccordement sur la bague 31, mais aussi des broches mâles 76 qui viennent alors s'enficher dans les broches femelles 68 déjà décrites, afin d'assurer la connexion électrique avec chacune.

## Revendications

1. Ensemble d'appareillage médical, permettant des connexions électriques à travers la peau, qui comporte un dispositif trans-cutané (3), comportant un socle (6) pourvu de moyens de fixation sur un os de l'organisme et des moyens de liaison étanche avec alternativement un bouchon temporaire (7) et un pilier trans-cutané (8) à l'intérieur duquel se situent des moyens de connexion électrique complémentaires de moyens associés de connexion électrique incorporés dans ledit socle, caractérisé en ce que ledit socle comporte une membrane (34) de protection étanche bactériologique desdits moyens incoporés dans ledit socle, perforable par au moins des broches électriquement conductrices (30, 68) que comporte ledit pilier (8), lors de sa mise en place en remplacement dudit bouchon temporaire (7).

2. Ensemble d'appareillage médical selon la revendication 1, caractérisé en ce que ledit socle comporte une base (16) présentant des ailes en bout desquelles sont prévus lesdits moyens de fixation sur l'os, ainsi qu'un bras (14, 52) enfermant des conducteurs électriques (19) en liaison électrique individuellement avec des broches (24) d'une fiche femelle de connexion électrique incorporée dans ledit socle (6), pour coopération avec des broches (30) d'une fiche mâle propre audit pilier (8).

3. Ensemble d'appareillage médical selon la revendication 1, caractérisé en ce que ledit socle comporte une base (16) présentant des ailes en bout desquelles sont prévus lesdits moyens de fixation sur l'os, ainsi qu'un bras (52) enfermant des conducteurs électriques (19) en liaison électrique individuellement avec des broches (68) de connexion électrique entre ledit socle (6)et ledit pilier (8).

4. Ensemble d'appareillage médical selon la revendication 2 ou 3, caractérisé en ce que lesdits conducteurs sont au moins partiellement sous forme de circuits imprimés noyés dans une platine enfermée dans ledit bras.

5. Ensemble d'appareillage médical selon l'une quelconque des revendications 1 à 4, caractérisé en ce que lesdits moyens de connexion électrique comportent une fiche femelle présentant une cannelure visible à travers ladite membrane pour y engager une broche de positionnement radial que comporte une fiche mâle desdits moyens de connexion électrique.

6. Ensemble d'appareillage médical selon l'une quelconque des revendications 1 à 5, caractérisé en ce que ladite fiche mâle est formée par un prolongateur coulissant dans une bague dudit pilier, ledit prolongateur formant également une fiche femelle accessible pour branchement d'unités de traitement de signaux électriques transmis par lesdits conducteurs.

7. Ensemble d'appareillage médical selon l'une quelconque des revendications 1 à 6, caractérisé en ce qu'il est associé audit dispositif trans-cutané en un ou plusieurs capteurs et/ou récepteurs implantés en sous-cutané dans ledit organisme.

8. Ensemble d'appareillage médical suivant la revendication 7, caractérisé par un capteur sous-cutané comportant une vis (43) d'implantation dans l'os présentant un alésage fileté axial pour recevoir une vis de couverture (44) et des moyens de coincement en contact conducteur pour l'extrémité d'un fil de liaison avec un circuit correspondant de l'implant trans-cutané.

9. Ensemble d'appareillage médical selon la revendication 8, caractérisé en ce que ledit capteur sous-cutané comporte une rondelle anti-dévissage avec une rainure (47) pour le passage dudit fil (29).

10. Ensemble d'appareillage médical selon l'une quelconque des revendications 1 à 9, caractérisé en ce qu'il est associé audit implant trans-cutané des capteurs implantés en sous-cutané sur un os dudit organisme, chacun étant en liaison électrique avec l'une des fiches électriquement conductrices de ladite fiche femelle par l'intermédiaire d'un fil courant sous la peau, ainsi qu'avec un connecteur extérieur amovible de transmission des informations sous forme de signaux électriques en provenance desdits capteurs vers une unité de traitement propre à établir des électroencéphalogrammes, électrocardiogrammes ou électromyogrammes.

11. Ensemble d'appareillage médical selon l'une quelconque des revendications précédentes, caractérisé en ce que lorsqu'on enlève ledit bouchon temporaire (7) dudit socle (6), ladite fiche femelle reste protégée par ladite membrane jusqu'à ce que celle-ci soit perforée quand on remplace ledit bouchon par le pilier transcutané(8) équipé de la fiche mâle.

12. Ensemble d'appareillage médical selon l'une quelconque des revendications précédentes, caractérisé en ce que ladite fiche femelle est constituée par des broches (68) montées dans une entretoise (67) d'une bague (31) dudit pilier (8)

13. Ensemble d'appareillage médical suivant la revendication 12, caractérisé en ce que ladite entretoise (65) présente des trous (67) pour le contact électrique entre lesdites broches (68) et des fils et/ou conduits conducteurs implantés dans l'organisme, à travers une platine (53) montée dans ledit socle (6).

## Patentansprüche

1. Medizinische Apparatur, die elektrische Verbindungen durch die Haut hindurch ermöglicht, mit einer Transcutaneinheit (3) mit einem Sockel (6), der mit Mitteln zur Befestigung an einem Knochen des Organismus und mit Verbindungsmitteln versehen ist, zur dichten wechselweisen Verbindung mit einem temporären Stopfen (7) und einem Transcutanpfosten (8), in dessen Innerem sich elektrische Verbindungsmittel befinden, die zu zugehörigen elektrischen Verbindungsmitteln in dem genannten Sockel komplementär sind, dadurch **gekennzeichnet**, daß der Sockel eine dichte bakteriologische Schutzmembran (34) für die in dem Sockel untergebrachten Mittel aufweist, welche Schutzmembran zumindest von elektrisch leitfähigen Stiften (30, 68) des Pfostens (8) durchstochen werden kann, wenn dieser anstelle des temporären Stopfens (7) eingesetzt wird.

2. Apparatur nach Anspruch 1, dadurch **gekennzeichnet**, daß der genannte Sockel eine Grundplatte (16), die Flügel bildet, an deren Ende die genannten Mittel zur Befestigung am Knochen vorgesehen sind, sowie einen Arm (14, 52) aufweist, der elektrische Leiter (19) umschließt, die einzeln elektrisch mit Stiften (24) einer in dem Sockel (6) ausgebildeten elektrischen Steckverbinderbuchse verbunden sind, zum Zusammenwirken mit Stiften (30) eines eigentlichen Stekkers an dem genannten Pfosten (8).

3. Apparatur nach Anspruch 1, dadurch **gekennzeichnet**, daß der Sockel eine Grundplatte (16), die Flügel bildet, an deren Ende die genannten Mittel zur Befestigung am Knochen vorgesehen sind, sowie einen Arm (52) aufweist, der elektrische Leiter (19) umschließt, die einzeln elektrisch mit Stiften (68) zur elektrischen Verbindung des Sockels (6) mit dem genannten Pfosten (8) verbunden sind.

4. Apparatur nach Anspruch 2 oder 3, dadurch **gekennzeichnet**, daß die genannten Leiter wenigstens zum Teil die Form von gedruckten Schaltungen haben, die in eine in dem genannten Arm aufgenommene Platine eingelassen sind.

5. Apparatur nach einem der Ansprüche 1 bis 4, dadurch **gekennzeichnet**, daß die genannten elektrischen Verbindungsmittel eine Steckbuchse umfassen, die eine durch die genannte Membran hindurch sichtbare Riffelung aufweist, für den Eingriff eines zur radialen Positionierung dienenden Stiftes eines Steckers der genannten elektrischen Verbindungsmittel.

6. Apparatur nach einem der Ansprüche 1 bis 5, dadurch **gekennzeichnet**, daß der genannte Stecker durch ein Verlängerungsstück gebildet wird, das in einem Ring des genannten Pfostens gleitet, wobei das Verlängerungsstück auch eine Steckbuchse bildet, die zugänglich ist für den Anschluß von Verarbeitungseinheiten zur Verarbeitung von elektrischen Signalen, die über die genannten Leiter übermittelt werden.

7. Apparatur nach einem der Ansprüche 1 bis 6, dadurch **gekennzeichnet**, daß der genannten Transcutaneinrichtung ein oder mehrere Aufnehmer und/ oder Empfänger zugeordnet sind, die subcutan in den Organismus implaritiert sind.

8. Apparatur nach Anspruch 7, **gekennzeichnet** durch einen subcutanen Aufnehmer, der eine Schraube (43) zur Implantation in den Knochen aufweist, die eine axiale Gewindebohrung zur Aufnahme einer Verschlußschraube (44) und Mittel zur leitenden Kontaktklemmung für das Ende einer Ader zur Verbindung mit einer entsprechenden Schaltung des Transcutanimplantats aufweist.

9. Apparatur nach Anspruch 8, dadurch **gekennzeichnet**, daß der genannte subcutane Aufnehmer eine Scheibe zur Sicherung der Verschraubung aufweist, mit einer Nut (47) für den Durchgang der genannten Ader (29).

10. Apparatur nach einem der Ansprüche 1 bis 9, dadurch **gekennzeichnet**, daß dem genannten Transcutanimplantat subcutan an einem Knochen des Organismus implantierte Aufnehmer zugeordnet sind, von denen jeder über eine unter der Haut verlaufende Ader elektrisch mit einer der elektrisch leitfähigen Klemme der genannten Steckbuchse verbunden ist, sowie mit einem lösbaren äußeren Verbinder zur Übertragung von Informationen in der Form von elektrischen Signalen, die von den genannten Aufnehmern stammen, zu einer eigentlichen Verarbeitungseinheit zur Bildung von Elektroencephalogrammen, Elektrocardiogrammen oder Elektromyogrammen.

11. Apparatur nach einem der vorstehenden Ansprüche, dadurch **gekennzeichnet**, daß, wenn der genannte temporäre Stopfen (7) vom Sockel (6) abgenommen wird, die genannte Steckbuchse durch die genannte Membran geschützt bleibt, bis diese perforiert wird, wenn man den genannten Stopfen durch den mit dem Stecker versehenen Transcutanpfosten (8) ersetzt.

12. Apparatur nach einem der vorstehenden Ansprüche, dadurch **gekennzeichnet**, daß die genannte Steckbuchse durch Stifte (68) gebildet wird, die in einer Zwischenwand (67) eines Ringes (31) des genannten Pfostens (8) montiert sind.

13. Apparatur nach Anspruch 12, dadurch **gekennzeichnet**, daß die genannte Zwischenwand (65) Löcher (67) aufweist, für den elektrischen Kontakt zwischen den genannten Stiften (68) und den Adern und/oder in den Organismus implantierten Leitern, durch eine in dem genannten Sockel (6) montierte Platine (53) hindurch.

## Claims

1. Set of medical apparatus, for providing electrical connections through the skin, which comprises a transcutaneous device (3) comprising a base (6) provided with means for attachment to a bone in the body and means for leaktight connection alternately to a temporary stopper (7) and to a transcutaneous pillar (8) inside which are electrical connecting means complementary to associated electrical connecting means incorporated in said base, characterised in that the base has a leaktight bacteriological protective membrane (34) for said means incorporated in said base, which is adapted to be perforated by at least one of the electrically conductive pins (30, 68) provided on said pillar (8) when it is put into position to replace said temporary stopper (7).

2. Set of medical apparatus according to claim 1, characterised in that the base comprises a bottom part (16) having lugs at the end of which are provided said means of attachment to the bone, as well as an arm (14, 52) containing electric conductors (19) which are individually electrically connected to the pins (24) of a female connector for electrical connection incorporated in said base (6), for co-operating with the pins (30) of a male plug belonging to said pillar (8).

3. Set of medical apparatus according to claim 1, characterised in that the base comprises a bottom part (16) having lugs at the end of which are provided said means of attachment to the bone, as well as an arm (52) containing electric conductors (19) which are individually electrically connected to pins (68) for electrically connecting said base (6) to said pillar (8).

4. Set of medical apparatus according to claim 2 or 3, characterised in that the conductors are at least partially in the form of printed circuits embedded in a plate enclosed in said arm.

5. Set of medical apparatus according to any one of claims 1 to 4, characterised in that the said electrical connecting means comprise a female socket having a channel which is visible through said membrane for the insertion of a radial positioning pin carried by a male plug of said electrical connecting means.

6. Set of medical apparatus according to any one of claims 1 to 5, characterised in that the male plug is formed by an extender sliding in a ring of said pillar, said extender also forming a female connector which is accessible for plugging in processing units for electrical signals transmitted by said conductors.

7. Set of medical apparatus according to any one of claims 1 to 6, characterised in that it is associated with said transcutaneous device at one or more pick-ups and/or receptors implanted subcutaneously in said body.

8. Set of medical apparatus according to claim 7, characterised by a subcutaneous pick-up comprising a screw (43) for implantation in the bone, having a threaded axial bore for receiving a cover screw (44) and means for jamming the end of a connecting wire into conductive contact with a corresponding circuit in the transcutaneous implant.

9. Set of medical apparatus according to claim 8, characterised in that the subcutaneous pick-up comprises an anti-slackening washer with a groove (47) to accommodate the wire (29).

10. Set of medical apparatus according to any one of claims 1 to 9, characterised in that it is associated with said transcutaneous implant of the pick-ups implanted subcutaneously on a bone in said body, each being electrically connected to one of the electrically conductive plugs of said female connector via a wire running under the skin, and with a removable external connector for transmitting data in the form of electrical signals coming from said pick-ups to a processing unit capable of producing electroencephalograms, electro-cardiograms or electro-myograms.

11. Set of medical apparatus according to any one of the preceding claims, characterised in that when the temporary stopper (7) is removed from said base (6), the female connector remains protected by said membrane until it is perforated when the stopper is replaced by the transcutaneous pillar (8) fitted with the male plug.

12. Set of medical apparatus according to any one of the preceding claims, characterised in that the female connector consists of pins (68) mounted in a spacer (67) of a ring (31) of said pillar (8).

13. Set of medical apparatus according to claim 12, characterised in that the spacer (65) has holes (67) for electrical contact between said pins (68) and wires and/or conductive conduits implanted in the body, through a plate (53) mounted in the base (6).
